(19) Europäisches Patentamt
European Patent Office
Office européen des brevets

(11) Veröffentlichungsnummer: **0 025 014**
**A1**

(12) # EUROPÄISCHE PATENTANMELDUNG

(21) Anmeldenummer: **80810244.6**

(22) Anmeldetag: **04.08.80**

(51) Int. Cl.[3]: **C 07 D 307/86**
C 07 D 317/24, C 07 D 317/46
C 07 C 145/04, C 07 C 149/437
A 01 N 47/24

(30) Priorität: **10.08.79 CH 7372/79**
**27.06.80 CH 4962/80**

(43) Veröffentlichungstag der Anmeldung:
**11.03.81 Patentblatt 81/10**

(84) Benannte Vertragsstaaten:
**AT BE CH DE FR GB IT LI NL**

(71) Anmelder: **CIBA-GEIGY AG**
**Patentabteilung Postfach**
**CH-4002 Basel(CH)**

(72) Erfinder: **Drabek, Jozef, Dr.**
**Benkenstrasse 12**
**CH-4104 Oberwil(CH)**

(72) Erfinder: **Böger, Manfred**
**Lindenstrasse 33**
**D-7858 Weil am Rhein 5(DE)**

(54) **N-(2-isobutyronitrilsulfenyl)-N-methyl-carbamate, Verfahren zu ihrer Herstellung, ihre Verwendung in der Schädlingsbekämpfung und sie enthaltende Schädlingsbekämpfungsmittel.**

(57) N-(2-Isobutyronitrilsulfenyl) -N-methylcarbamate der Formel

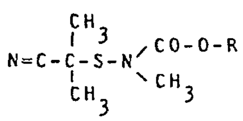 (1),

worin R 2,2-Dimethyl-(2H, 3H) -dihydrobenzofuran-7-yl, α-Naphthyl,

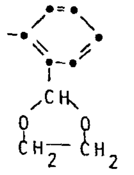 , 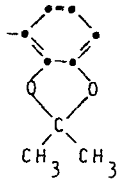 oder 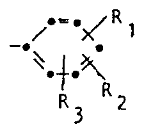 und

$R_1$, $R_2$ und $R_3$ je Wasserstoff, $C_1$-$C_5$-Alkyl, $C_1$-$C_5$-Alkoxy oder $C_1$-$C_5$-Alkylthio bedeuten. Verfahren zur Herstellung dieser Verbindungen und deren Verwendung zur Bekämpfung von Schädlingen an Tieren und Pflanzen sind beschrieben.

EP 0 025 014 A1

CIBA-GEIGY AG 5-12469/1+2

Basel (Schweiz)

BEZEICHNUNG GEÄNDERT
siehe Titelseite

N-(2-Isobutyronitrilsulfenyl)-N-methylcarbamate, Verfahren zu ihrer Herstellung und ihre Verwendung in der Schädlingsbekämpfung

Die vorliegende Erfindung betrifft neue N-(2-Isobutyronitrilsulfenyl)-N-methylcarbamate, welche eine Wirkung gegen Schädlinge besitzen, und Verfahren zu ihrer Herstellung sowie Schädlingsbekämpfungsmittel, welche die vorstehend genannten Carbamate als aktive Komponente enthalten, und Verfahren zur Bekämpfung von Schädlingen unter Verwendung der neuen Verbindungen.

Die N-(2-Isobutyronitrilsulfenyl)-N-methylcarbamate entsprechen der Formel I

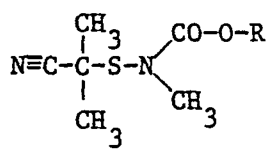 (I),

worin R 2,2-Dimethyl-(2H,3H)-dihydrobenzofuran-7-yl, α-Naphthyl,

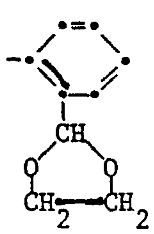 , 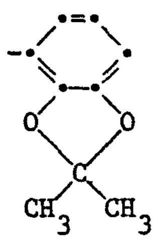 oder 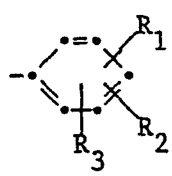 und

$R_1$, $R_2$ und $R_3$ je Wasserstoff, $C_1$-$C_5$-Alkyl, $C_1$-$C_5$-Alkoxy oder $C_1$-$C_5$-Alkylthio bedeuten.

Die für $R_1$, $R_2$ und $R_3$ in Frage kommenden Alkyl-, Alkoxy- und Alkylthiogruppen können geradktettig oder verzweigt sein. Beispiele solcher Gruppen sind u.a.: Methyl, Methoxy, Methylthio, Aethyl, Aethoxy, Propyl, Propoxy, Isopropyl, Isopropoxy, n-, i-,

sek.-, tert.Butyl, n-Pentyl und dessen Isomere.

Wegen ihrer Wirkung bevorzugt sind Verbindungen der Formel I, worin R 2,2-Dimethyl-(2H,3H)-dihydrobenzofuran-7-yl-, α-Naphthyl,

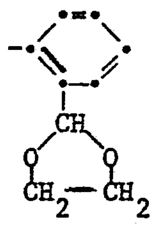 , 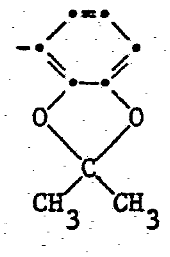 oder 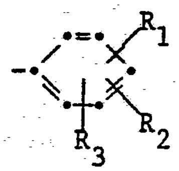 und

$R_1$ Wasserstoff oder $C_1$-$C_5$-Alkyl und $R_2$ und $R_3$ je Wasserstoff, Methyl, Isopropoxy oder Methylthio bedeuten.

Die Verbindungen der Formel I werden analog bekannten Verfahren hergestellt, indem man z.B.

a) eine Verbindung der Formel II

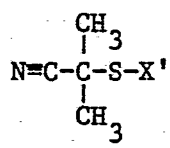 (II)

in Gegenwart einer Base mit einer Verbindung der Formel III

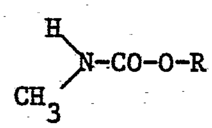 (III)

umsetzt; oder

b) dass man eine Verbindung der Formel IV

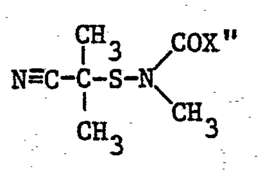 (IV)

in Gegenwart einer Base mit einer Verbindung der Formel V

HO-R (V)

umsetzt, wobei in den Formeln II bis V R die für Formel I bereits angegebene Bedeutung hat und X' für ein Halogenatom, insbesondere ein Chloratom, und X" für ein Halogenatom, insbesondere ein Fluoratom, steht.

Die Verfahren a) und b) werden bei einer Reaktionstemperatur zwischen -50°C und +130°C, vorzugsweise zwischen -10°C und +100°C, bei normalem oder leicht erhöhtem Druck und in Gegenwart eines gegenüber den Reaktionsteilnehmern inerten Lösungs- oder Verdünnungsmittels vorgenommen.

Als für diese Verfahren geeignete Basen kommen insbesondere tertiäre Amine, wie Trialkylamine, Pyridine und Dialkylaniline, ferner Hydroxide, Oxide, Carbonate und Bicarbonate von Alkali- und Erdalkalimetallen sowie Alkalimetallalkoholate, wie z.B. Kalium-tert.butylat und Natrium-methylat, in Betracht.

Als Lösungs- oder Verdünnungsmittel eignen sich z.B. Aether und ätherartige Verbindungen wie Diäthyläther, Di-iso-propyläther, Dioxan, Tetrahydrofuran; aliphatische und aromatische Kohlenwasserstoffe, insbesondere Benzol, Toluol, Xylole; und Ketone wie Aceton, Methyläthylketon und Cyclohexanon.

Die in den vorstehend aufgeführten Verfahren verwendeten Ausgangsstoffe der Formeln II, III und V sind bekannt bzw. können analog zu den bekannten Methoden hergestellt werden.

Die Ausgangsstoffe der Formel IV sind bekannt und können aus bekannten Vorläufern leicht erhalten werden, indem man z.B. eine Verbindung der bereits definierten Formel II in Gegenwart einer

Base mit einer Verbindung der Formel VI

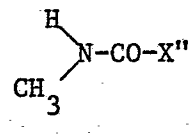 (VI)

umsetzt, worin X" die bereits angegebene Bedeutung hat.

Das Verfahren zur Herstellung der Ausgangsstoffe der Formel IV wird zweckmässig in Gegenwart eines gegenüber den Reaktionsteilnehmern inerten Lösungs- oder Verdünnungsmittels, bei einer Reaktionstemperatur von -50°C bis +130°C, unter normalem Druck durchgeführt. Als für dieses Verfahren geeignete Basen und Lösungsmittel kommen die für die oben aufgeführten Verfahren a) und b) bereits erwähnten Substanzen in Betracht.

Die Verbindungen der Formel I eignen sich zur Bekämpfung von verschiedenartigen Schädlingen an Tieren und Pflanzen.

Insbesondere eignen sich die Verbindungen der Formel I zur Bekämpfung aller Entwicklungsstadien von Insekten, phytopathogenen Milben sowie von Zecken, z.B. der Ordnungen Lepidoptera, Coleoptera, Homoptera, Heteroptera, Diptera, Acarina, Thysanoptera, Orthoptera, Anoplura, Siphonaptera, Mallophaga, Thysanura, Isoptera, Psocoptera und Hymenoptera.

Darüber hinaus besitzen die Verbindungen der Formel I eine wertvolle Wirkung gegen pflanzenparasitäre Nematoden sowie gegen Akarinen, insbesondere ektoparasitäre Akarinen (Milben und Zecken) z.B. der Familien Ixodidae, Argasidae und Dermanyssidae.

Vor allem eignen sich Verbindungen der Formel I zur Bekämpfung von pflanzenschädigenden Insekten, insbesondere pflanzenschädigenden Frassinsekten, in Zier- und Nutzpflanzen, insbesondere in Baumwollkulturen (z.B. gegen Spodoptera littoralis und Heliothis

virescens), Gemüsekulturen (z.B. gegen Leptinotarsa decemlineata und Myzus persicae) und Reiskulturen (z.B. gegen Reiszikaden).

Wirkstoffe der Formel I zeigen auch eine sehr günstige Wirkung gegen Fliegen, wie z.B. Musca domestica und Mückenlarven. Daneben zeichnen sich die Verbindungen der Formel I durch eine breite ovizide und ovilarvizide Wirkung aus.

Die Verbindungen der Formel I können in der Regel entweder in unveränderter Form oder zusammen mit den in der Formulierungstechnik üblichen Hilfsmitteln als Formulierungen, wie z.B. Emulsionskonzentrate, Suspensionskonzentrate, direkt versprühbare oder verdünnbare Lösungen, verdünnte Emulsionen, Spritzpulver, lösliche Pulver, Stäubemittel, Granulate, auch Feinstverkapselungen in polymeren Stoffen und dergleichen, in bekannter Weise eingesetzt werden. Die Anwendungsformen wie Versprühen, Vernebeln, Verstäuben, Verstreuen oder Giessen, richten sich ganz nach den Verwendungszwecken. Dabei ist zu beachten, dass durch die Applikationsmethode sowie die Art und Menge der für die Zubereitung der Formulierung verwendeten Hilfsmaterialien das biologische Verhalten des Wirkstoffes der Formel I nicht wesentlich beeinflusst wird.

Die Formulierungen werden in bekannter Weise hergestellt, z.B. durch inniges Vermischen und/oder Vermahlen der Wirkstoffe mit Streckmitteln, also mit Lösungsmitteln, festen Trägerstoffen, und gegebenenfalls unter Verwendung oberflächenaktiver Substanzen (Tenside). Als Lösungsmittel können in Frage kommen: Aromatische Kohlenwasserstoffe, bevorzugt die Fraktionen $C_8$ bis $C_{12}$, d.h. Xylolgemische bis hin zu substituierten Naphthalinen, aliphatische Kohlenwasserstoffe wie Cyclohexan oder Paraffine, Alkohole und Glykole sowie deren Aether und Ester, stark polare Lösungsmittel wie Dimethylsulfoxid oder Dimethylformamid, sowie Wasser. Als feste Trägerstoffe, z.B. für Stäubemittel und dispergierbare Pulver,

werden meist natürliche Gesteinsmehle verwendet. Chemisch kommen hier vor allem Calcit, Talkum, Kaolinit, Montmorillonit oder Attapulgit in Frage. Zur Verbesserung der physikalischen Eigenschaften können auch hochdisperse Kieselsäuren oder hochdisperse saugfähige Polymere zugesetzt werden. Als gekörnte Granulatträger kommen poröse Typen wie z.B. Bims, Ziegelbruch, Sepiolit und Bentonit, als nicht sorptive Träger Materialien wie z.B. Calcit oder Sand in Frage. Darüberhinaus kann eine Vielzahl von vorgranulierten Materialien anorganischer oder organischer Natur, vom Dolomit bis hin zu zerkleinerten Nussschalen oder Maiskolben verwendet werden.

Als oberflächenaktive Substanzen kommen je nach der Polarität des zu formulierenden Wirkstoffes der Formel I nichtionogene, kation- und/oder anionaktive Tenside mit guten Emulgier-, Dispergier- und Netzeigenschaften in Betracht.

Geeignete anionaktive Tenside sind beispielsweise quaternäre Ammoniumverbindungen, wie z.B. Cetyltrimethyl-ammonium-bromid. Geeignete anionaktive Tenside sind z.B. Seifen, Salze von aliphatischen Monoestern der Schwefelsäure, wie z.B. Natrium-laurylsulfat, Salze von sulfonierten aromatischen Verbindungen, wie z.B. Natriumdodecylbenzolsulfonat, Natrium-, Calcium- und Ammoniumligninsulfonat, Butylnaphthalinsulfonat und ein Gemisch aus den Natriumsalzen von Diisopropyl- und Triisopropylnaphthalinsulfonat. Geeignete nichtionogene Tenside sind beispielsweise die Kondensationsprodukte von Aethylenoxid mit Fettalkoholen, wie z.B. Oleylalkohol oder Cetylalkohol, oder mit Alkylphenolen, wie z.B. Octylphenol, Nonylphenol und Octylkresol.Andere nicht-ionische Mittel sind die Teilester, die sich von langkettigen Fettsäuren und Hexitanhydriden ableiten, die Kondensationsprodukte dieser Teilester mit Aethylenoxid und die Lecithine.

Die in der Formulierungstechnik gebräuchlichen nichtionogenen, anion- und kationaktiven Tenside sind u.a. in folgenden Publikationen beschrieben:

"Mc Cutcheon's Detergents and Emulsifiers Anual" MC Publishing Corp., Ringewood, New Jersey,

Sisely and Wood, "Encyclopedia of Surface Active Agents", Chemical Publishing Co., Inc., New York.

Die Formulierungen enthalten in der Regel 0,1 bis 99% , insbesondere 0,1 bis 95%, Wirkstoff der Formel I und wenigstens 0 bis 25% eines Tensides sowie 1 bis 99,9% eines festen oder flüssigen Zusatzstoffes.

Die Formulierungen können auch Mittel wie Stabilisatoren, Entschäumer, Viskositätsregulatoren, Bindemittel, Haftmittel sowie auch Düngerzusätze zur Erzeugung spezieller Effekte enthalten.

Die Wirkstoffe der Formel I können beispielsweise wie folgt formuliert werden: (Angaben in Gewichtsprozenten)

<u>Formulierungsbeispiele für flüssige Wirkstoffe der Formel I</u>

<u>Emulsions-Konzentrate</u>

| | | |
|---|---|---|
| a) | Wirkstoff | 20% |
| | Ca-Dodecylbenzolsulfonat | 5% |
| | Ricinusöl-polyglykoläther (36 Mol AeO) | 5% |
| | Xylolgemisch | 70% |
| b) | Wirkstoff | 40% |
| | Ca-Dodecylbenzolsulfonat | 8% |
| | Tributylphenol-polyglykoläther (30 Mol AeO) | 12% |
| | Cyclohexanon | 15% |
| | Xylolgemisch | 25% |

| | | |
|---|---|---|
| c) | Wirkstoff | 50% |
| | Tributylphenol-Polyglykoläther | 4,2% |
| | Calcium-Dodecylbenzolsulfonat | 5,8% |
| | Cyclohexanon | 20% |
| | Xylolgemisch | 20% |

Aus solchen Konzentraten können durch Verdünnen mit Wasser Emulsionen jeder gewünschten Konzentration hergestellt werden.

Lösungen

| | | |
|---|---|---|
| a) | Wirkstoff | 80% |
| | Aethylenglykol-monomethyläther | 20% |
| b) | Wirkstoff | 10% |
| | Polyäthylenglykol 400 | 70% |
| | N-Methyl-2-pyrrolidon | 20% |
| c) | Wirkstoff | 5% |
| | Epoxydiertes Pflanzenöl | 1% |
| | Benzin (Siedegrenzen 160-190°C) | 94% |
| d) | Wirkstoff | 95% |
| | Epoxydiertes Pflanzenöl | 5% |

Diese Lösungen sind zur Anwendung in Form kleinster Tropfen geeignet.

Granulate

| | | |
|---|---|---|
| a) | Wirkstoff | 5% |
| | Kaolin 0.2-0,8 mm | 94% |
| | Hochdisperse Kieselsäure | 1% |
| b) | Wirkstoff | 10% |
| | Attaclay 0.3-1 mm | 90% |

Der Wirkstoff wird in Methylenchlorid gelöst, auf den Träger aufgesprüht und das Lösungsmittel anschliessend im Vakuum abgedampft.

Stäubemittel

| | | |
|---|---|---|
| a) | Wirkstoff | 2% |
| | Hochdisperse Kieselsäure | 1% |
| | Talkum | 97% |
| b) | Wirkstoff | 5% |
| | Hochdisperse Kieselsäure | 5% |
| | Kaolin feinteilig | 90% |

Durch inniges Vermischen der Trägerstoffe mit dem Wirkstoff erhält man gebrauchsfertige Stäubemittel.

Formulierungsbeispiele für feste Wirkstoffe der Formel I

Spritzpulver

| | | |
|---|---|---|
| a) | Wirkstoff | 20% |
| | Na-Ligninsulfonat | 5% |
| | Na-Laurylsulfat | 3% |
| | Kieselsäure | 5% |
| | Kaolin | 67% |
| b) | Wirkstoff | 60% |
| | Na-Ligninsulfonat | 5% |
| | Na-Diisobutylnaphthalinsulfonat | 6% |
| | Octylphenolpolyglykoläther (7-8 Mol AeO) | 2% |
| | Hochdisperse Kieselsäure | 27% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration

verdünnen lassen.

Emulsions-Konzentrat

| | |
|---|---|
| Wirkstoff | 10% |
| Octylphenolpolyglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

Stäubemittel

| | | |
|---|---|---|
| a) | Wirkstoff | 5% |
| | Talkum | 95% |
| b) | Wirkstoff | 8% |
| | Kaolin feinteilig | 92% |

Man erhält anwendungsfertige Stäubemittel, indem der Wirkstoff mit den Trägern vermischt und auf einer geeigneten Mühle vermahlen wird.

Extruder Granulat

| | |
|---|---|
| Wirkstoff | 10% |
| Na-Ligninsulfonat | 2% |
| Carboxymethylcellulose | 1% |
| Kaolin feinteilig | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

Umhüllungs-Granulat

| | |
|---|---|
| Wirkstoff | 3% |
| Polyäthylenglykol 200 | 3% |
| Kaolin (0.3-0.8 mm) | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

Suspensions-Konzentrat

| | |
|---|---|
| Wirkstoff | 40% |
| Aethylenglykol | 10% |
| Nonylphenolpolyglykoläther (15 Mol AeO) | 6% |
| Na-Ligninsulfonat | 10% |
| Carboxymethylcellulose | 1% |
| Formalin (37%ige Formaldehyd-Lösung) | 0.2% |
| Silikonöl in Form einer 75%igen Emulsion | 0.8% |
| Leitungswasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1: Herstellungsverfahren

a) Herstellung von (2-Fluorcarbonyl-4-cyano-4-methyl)-2-aza-4-sulfapentan (Ausgangsstoff)

Zu einer Lösung von 11,7 g wasserfreier Fluorwasserstoffsäure in 100 ml Toluol wurden bei einer Temperatur von -50°C unter Rühren 34,8 ml Methylisocyanat zugegeben und das Reaktionsgemisch wurde während 2 Stunden nachgerührt. Zu der auf diese Weise erhaltenen Lösung wurden 79,4 g Isobutyronitril-2-sulfenylchlorid zugegeben und anschliessend unter Rühren bei einer Temperatur von -50°C bis -20°C 80,85 ml Triäthylamin zugetropft. Danach wurde das Reaktionsgemisch während 2 Stunden bei -20°C, 1 Stunde bei Raumtemperatur und zuletzt 1 Stunde bei 50°C gerührt. Nach dem Abnutschen des entstandenen Triäthylaminhydrochlorids wurde das Filtrat am Rotationsverdampfer eingeengt. Nach dem Abdestillieren des Rohproduktes am Hochvakuum erhielt man das (2-Fluorcarbonyl-4-cyano-4-methyl)-2-aza-4-sulfapentan der Formel

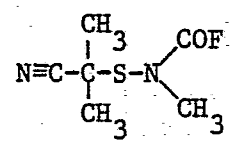

als eine gelbe Flüssigkeit (Kp. 78-80°C/0.25 m.b.).

b) Herstellung von N-(2-Isobutyronitrilsulfenyl)-(2,3-dihydro-2,2-dimethyl-7-benzofuranyl)-N-methylcarbamat (Endprodukt)

Zu einer Lösung von 5,5 g 2,3-Dihydro-2,2-dimethyl-7-hydroxybenzofuran in 80 ml Toluol wurden unter Rühren 5,95 ml Triäthylamin und anschliessend bei max. 30°C 5,9 g (2-Fluorcarbonyl-4-cyano-4-methyl)-2-aza-4-sulfa-pentan zugetropft. Das Reaktionsgemisch wurde 16 Stunden bei Raumtemperatur und anschliessend 2 1/2 Stunden bei 40-50°C nachgerührt, filtriert und eingeengt. Das Rohprodukt wurde in Diäthyläther aufgenommen, 4 mal mit 50 ml Wasser gewaschen, die

organische Phase über $Na_2SO_4$ getrocknet und zuletzt eingeengt. Auf diese Weise erhielt man das N-(2-Isobutyronitrilsulfenyl)-(2,3-dihydro-2,2-dimethyl-7-benzofuranyl)-N-methylcarbamat der Formel

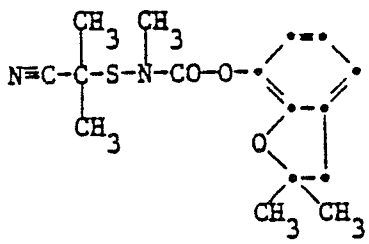

als ein gelbes Oel mit einer Brechungsindex von $n_D^{21°}$: 1,5293.

Die nachfolgenden Verbindungen können analog zu dem vorstehend beschriebenen Herstellungsverfahren hergestellt werden.

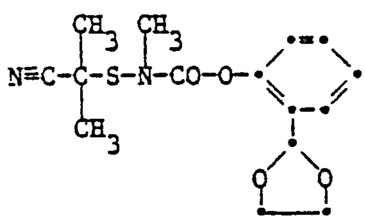

$n_D^{21°}$ : 1.5393

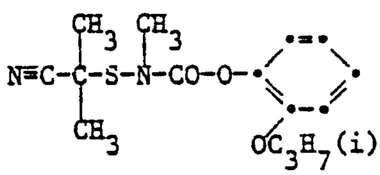

$n_D^{21°}$: 1,5201

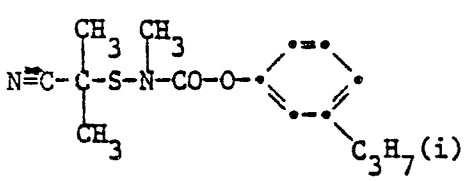

$n_D^{21°}$: 1.5231

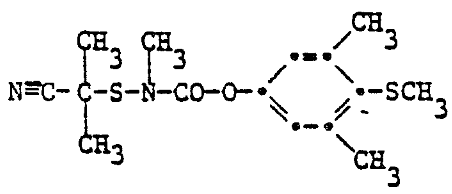

$n_D^{21°}$: 1,5513

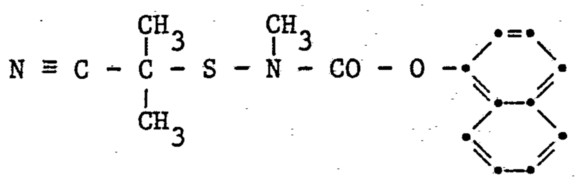

Smp.: 94-97°C

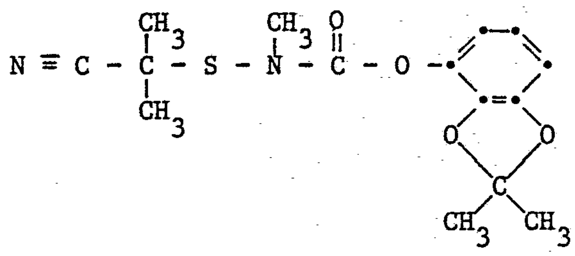

Smp.: 54-56°C

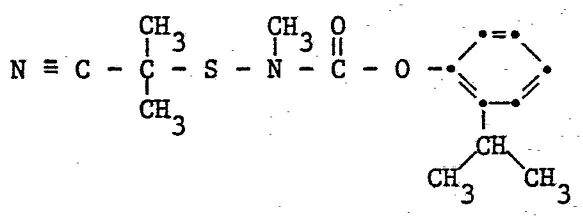

$n_D^{25°}$: 1,5210

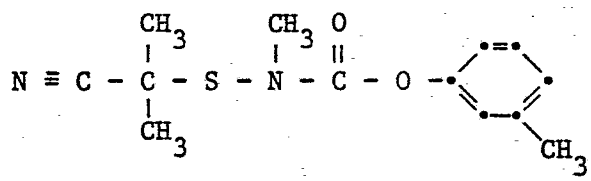

$n_D^{25°}$: 1,5285

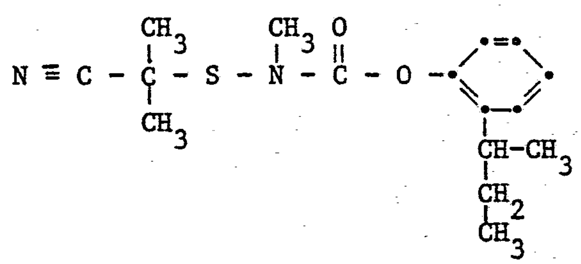

$n_D^{25°}$: 1,5179

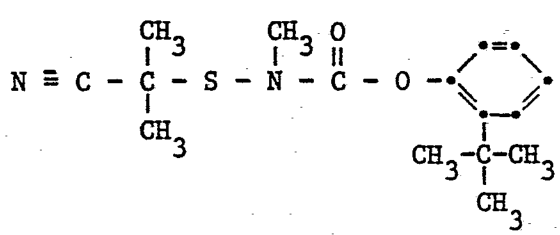

$n_D^{40°}$: 1,5208

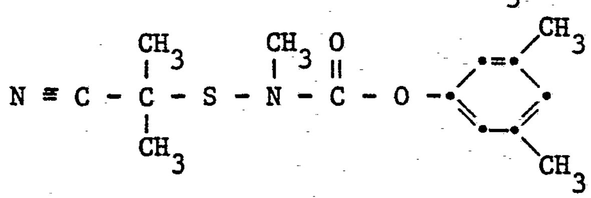

$n_D^{25°}$: 1,5271

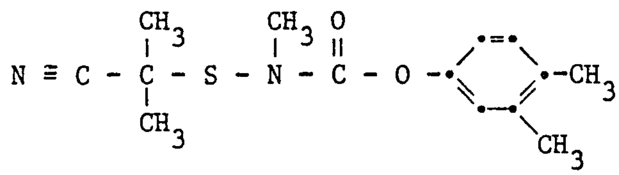

$n_D^{25°}$: 1,5300

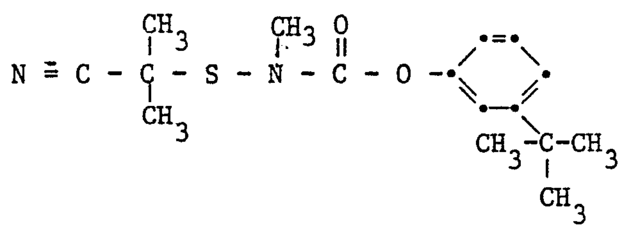

$n_D^{25°}$: 1,5209

Beispiel 2: Insektizide Frassgift-Wirkung: Spodoptera littoralis, Dysdercus fasciatus und Heliothis virescens

Baumwollpflanzen wurden mit einer Versuchslösung, enthaltend 50, 100, 200 oder 400 ppm der zu prüfenden Verbindung, besprüht.

Nach dem Antrocknen des Belages wurden die Pflanzen je mit Larven der Spezies Spodoptera littoralis (L3-Stadium) Dysdercus fasciatus (L4) oder Heliothis virescens (L3) besetzt. Man verwendete pro Versuchsverbindung und pro Test-Spezies zwei Pflanzen, und eine Auswertung der erzielten Abtötungsrate erfolgte nach 2, 4, 24 und 48 Stunden. Der Versuch wurde bei 24°C und 60% relativer Luftfeuchtigkeit durchgeführt.

Innerhalb der oben angegebenen Konzentrationsgrenzen zeigten Verbindungen gemäss dem Herstellungsbeispiel 1 100%ige Wirkung gegen Larven der Spezies Spodoptera littoralis, Dysdercus fasciatus und Heliothis virescens.

Beispiel 3: Insektizide Kontakt-Wirkung: Myzus persicae

In Wasser angezogene Pflanzen (Vicia fabae) wurden vor dem Versuchsbeginn je mit ca. 200 Individuen der Spezies Myzus persicae

besiedelt. Die so behandelten Pflanzen wurden 3 Tage später mit einer Lösung enthaltend 10 oder 1 ppm der zu prüfenden Verbindung aus 30 cm Distanz bis zur Tropfnässe besprüht. Man verwendete pro Test-Verbindung und pro Konzentration zwei Pflanzen, und eine Auswertung der erzielten Abtötungsrate erfolgte nach weiteren 24 Stunden.

Innerhalb der oben angegebenen Konzentrationsgrenzen zeigten Verbindungen gemäss Beispiel 1 eine 100%ige Wirkung gegen Insekten der Spezies Myzus persicae.

Beispiel 4: Insektizide systemische Wirkung: Aphis craccivora

Bewurzelte Bohnenpflanzen wurden in Töpfe, welche 600 ccm Erde enthalten, verpflanzt. Anschliessend wurden 50 ml einer Versuchslösung , 25 ppm, 5 ppm bzw. 1 ppm der zu prüfenden Verbindung direkt auf die Erde gegossen.

Nach 24 Stunden wurden auf die oberirdischen Pflanzenteile Blattläuse (Aphis craccivora) gesetzt und die Pflanzen mit einem unten zugeschnürten Plastikzylinder überstülpt, um die Läuse vor einer eventuellen Kontakt- oder Gaswirkung der Testsubstanz zu schützen.

Die Auswertung der erzielten Abtötung erfolgte 24 und 48 Stunden nach Versuchsbeginn. Pro Konzentrationsdosis Testsubstanz wurden zwei Pflanzen, je eine in einem separaten Topf, verwendet. Der Versuch wurde bei 25°C und 70% relativer Luftfeuchtigkeit durchgeführt.

Innerhalb der oben angegebenen Konzentrationsgrenzen zeigten Verbindungen gemäss Beispiel 1 eine 100%ige systemische Wirkung gegen Insekten der Spezies Aphis craccivora.

Patentansprüche

1. Ein N-(2-Isobutyronitrilsulfenyl)-N-methylcarbamat der Formel

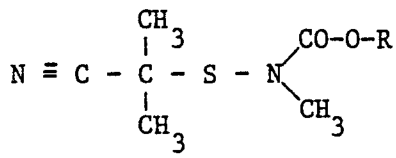

(I),

worin R 2,2-Dimethyl-(2H,3H)-dihydrobenzofuran-7-yl, α-Naphthyl,

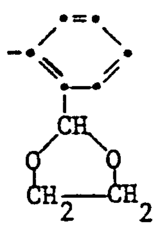

,

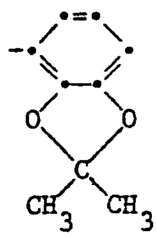

oder

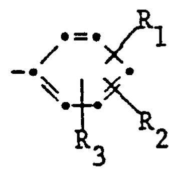

und

$R_1$, $R_2$ und $R_3$ je Wasserstoff, $C_1$-$C_5$-Alkyl, $C_1$-$C_5$-Alkoxy oder $C_1$-$C_5$-Alkylthio bedeuten.

2. Eine Verbindung gemäss Anspruch 1, worin R 2,2-Dimethyl-(2H,3H)-dihydrobenzofuran-7-yl, α-Naphthyl,

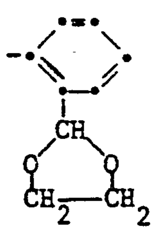

,

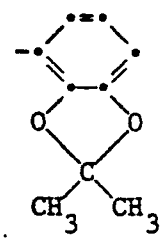

oder

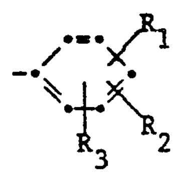

und

$R_1$ Wasserstoff oder $C_1$-$C_5$-Alkyl und $R_2$ und $R_3$ je Wasserstoff, Methyl, Isopropoxy oder Methylthio bedeuten.

3. Die Verbindung gemäss Anspruch 2 der Formel

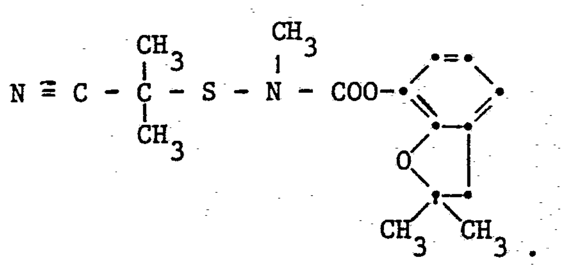

4. Die Verbindung gemäss Anspruch 2 der Formel

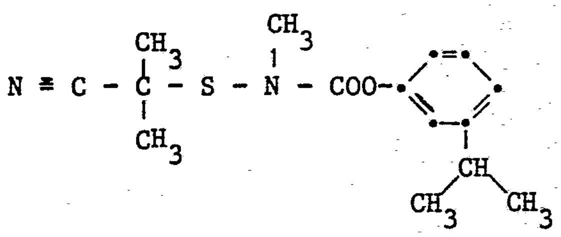

5. Verfahren zur Herstellung von Verbindungen gemäss Anspruch 1, dadurch gekennzeichnet, dass man

a) eine Verbindung der Formel

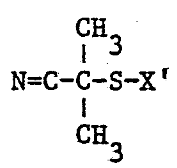

in Gegenwart einer Base mit einer Verbindung der Formel

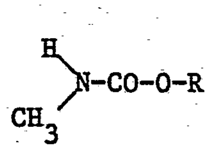

umsetzt; oder

b) dass man eine Verbindung der Formel

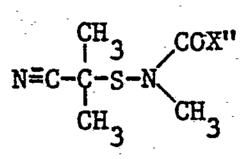

in Gegenwart einer Base mit einer Verbindung der Formel

$$HO-R$$

umsetzt, wobei R die im Anspruch 1 bereits angegebene Bedeutung hat und X' und X" je ein Halogenatom bedeuten.

6. Schädlingsbekämpfungsmittel, welches als aktive Komponente 0,1 bis 99,9% einer Verbindung gemäss Anspruch 1 und wenigstens 0 bis 25% eines Tensides sowie 1 bis 99,9% eines festen oder flüssigen Zusatzstoffes enthält.

7. Verwendung einer Verbindung gemäss Anspruch 1 zur Bekämpfung von Schädlingen an Tieren und Pflanzen.

8. Verwendung gemäss Anspruch 7 zur Bekämpfung von Insekten und Vertretern der Ordnung Akarina.

0025014

Europäisches Patentamt

# EUROPÄISCHER RECHERCHENBERICHT

Nummer der Anmeldung

EP 80810244.6

| EINSCHLÄGIGE DOKUMENTE | | | KLASSIFIKATION DER ANMELDUNG (Int Cl. ) |
|---|---|---|---|
| Kategorie | Kennzeichnung des Dokuments mit Angabe, soweit erforderlich, der maßgeblichen Teile | betrifft Anspruch | |
| | Keine Entgegenhaltungen. | | C 07 D 307/86<br>C 07 D 317/24<br>C 07 D 317/46<br>C 07 C 145/04<br>C 07 C 149/437<br>A 01 N 47/24 |
| | | | RECHERCHIERTE SACHGEBIETE (Int. Cl.³) |
| | | | C 07 D 307/00<br>C 07 D 317/00<br>C 07 C 149/00<br>A 01 N 47/00 |
| | | | KATEGORIE DER GENANNTEN DOKUMENTE |
| | | | X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| WIEN | 16-10-1980 | DASCHL |